# EUROPÄISCHE PATENTSCHRIFT

(11) **EP 3 668 831 B1**
(45) Veröffentlichungstag und Bekanntmachung des Hinweises auf die Patenterteilung: **23.06.2021**
(21) Anmeldenummer: 18752155.4
(22) Anmeldetag: 13.08.2018
(51) Int. Cl.: C07C 67/54, C07C 67/08, C07C 69/54, C12P 7/42

(54) **VERFAHREN ZUR KONTINUIERLICHEN HERSTELLUNG VON ACRYLSÄURE-N-BUTYLESTER ODER ACRYLSÄURE-ISOBUTYLESTER**
METHOD FOR THE CONTINUOUS PRODUCTION OF N-BUTYL ACRYLATE OR ISOBUTYL ACRYLATE
PROCÉDÉ DE FABRICATION EN CONTINU D'ACRYLATE DE N-BUTYLE OU D'ACRYLATE D'ISOBUTYLE

(30) Priorität: 17.08.2017 EP 17186691
(43) Veröffentlichungstag der Anmeldung: 24.06.2020
(73) Patentinhaber: BASF SE, 67056 Ludwigshafen am Rhein (DE)
(72) Erfinder: LANG, Ortmund, 67056 Ludwigshafen (DE); BLASCHKE, Tim, 2040 Antwerpen (BE); RAITH, Christian, 67056 Ludwigshafen (DE); SCHAFRANKA, Michael, 67056 Ludwigshafen (DE); HECHLER, Claus, 67056 Ludwigshafen (DE)
(74) Vertreter: BASF IP Association
(86) Internationale Anmeldenummer: PCT/EP2018/071863
(87) Internationale Veröffentlichungsnummer: WO 2019/034577

(56) Entgegenhaltungen:
- WO-A1-2012/071158
- WO-A2-03/082795

## Beschreibung

Die Erfindung betrifft ein Verfahren zur kontinuierlichen Herstellung eines Acrylsäurebutylesters H₂C=CH-C(=O)OR, mit R = n-Butyl oder iso-Butyl.

Verfahren zur Herstellung von Acrylsäurealkylestern aus 3-Hydroxy-propionsäure sind bekannt. Prinzipiell kann 3-Hydroxy-propionsäure zunächst in einem ersten Schritt mit einem Alkohol verestert und dann in einem nachfolgenden Schritt der resultierende 3-Hydroxypropionsäureester zum entsprechenden Acrylsäurealkylester dehydratisiert werden. Alternativ kann 3-Hydroxy-propionsäure auch zunächst in einem ersten Schritt dehydratisiert werden und dann in einem nachfolgenden Schritt die resultierende Acrylsäure mit einem Alkohol verestert werden.

WO 03/082795 A2 (Cargill, Inc.) beschreibt in Beispiel 13 (Seite 14) eine Eintopf-Synthese von n-Butylacrylat ausgehend von wässriger 3-Hydroxypropionsäure und n-Butanol (Katalysator: H₂SO₄). Das Verfahren erfolgt in drei Schritten: zunächst wird das Wasser vollständigt abdestilliert, dann wird in einer Destillationsapparatur n-Butanol abgetrennt und schließlich, nach Temperatur- und Druckerniedrigung, aus dem Rückstand das Produkt n-Butylacrylat abdestilliert. Die Ausbeute beträgt lediglich 37 %.

WO 2015/036218 A1 (BASF SE) beschreibt ein Verfahren zur Dehydratisierung von wässriger 3-Hydroxy-propionsäure zu Acrylsäure.

Die Veresterung von Acrylsäure mit einem Alkohol kann insbesondere nach den Verfahren gemäß DE 196 04 267 A1 (BASF AG) oder EP 765 859 A1 (BASF AG) erfolgen:
DE 196 04 267 A1 offenbart ein Verfahren zur kontinuierlichen Herstellung von Alkylestern der (Meth)acrylsäure wobei die Aufarbeitung in zwei miteinander verschalteten Rektifikationseinheiten erfolgt.
EP 765 859 A1 lehrt ein Verfahren zur kontinuierlichen Herstellung von Acrylsäurealkylestern bei dem die Reaktionszone aus einer Kaskade von mindestens zwei hintereinandergeschalteten Reaktionsbereichen besteht.

EP 1 182 189 B1 (Rohm and Haas Company) betrifft ein Verfahren zum fortlaufenden Wiedergewinnen von n-Butylacrylat aus einem Veresterungsreaktionsgemisch.

WO 2012/071158 A1 (Rohm and Haas Company) beschreibt ein spezielles Destillationsverfahren für C₁₋₄-Alkyl(meth)acrylate, wobei der verdampfte Reaktorinhalt direkt in eine Kolonne geleitet wird.

Ein möglicher Weg zur Herstellung von 3-Hydroxypropionsäure verläuft über Glukose durch Fermentation, wobei die Glukose aus nachwachsenden Rohstoffen wie zum Beispiel Mais gewonnen wird. Die Herstellung von 3-Hydroxypropionsäure durch Fermentation wird beispielsweise in WO 2012/074818 A2 (Novozymes, Inc.) beschrieben.

Der vorliegenden Erfindung lag die Aufgabe zugrunde, unter Überwindung von Nachteilen des Stands der Technik, ein verbessertes wirtschaftliches Verfahren zur Herstellung von Acrylsäure-n-butylester und Acrylsäure-iso-butylester bereitzustellen. Das Herstellverfahren sollte zudem besonders einfach zu realisieren und durchzuführen und zudem besonders wirtschaftlich sein.

Demgemäß wurde ein Verfahren zur kontinuierlichen Herstellung eines Acrylsäurealkylesters H₂C=CH-C(=O)OR, mit R = n-Butyl oder iso-Butyl, gefunden, welches dadurch gekennzeichnet ist, dass man wässrige 3-Hydroxy-propionsäure unter dehydratisierenden und veresternden Bedingungen in Gegenwart des entsprechenden Butanols R-OH in einem Reaktor mit Rektifikationskolonne umsetzt und gebildeten Acrylsäurebutylester, unumgesetztes Butanol sowie eingesetztes und gebildetes Wasser als ternäres Azeotrop über Kopf abdestilliert, nach Scheidung in eine jeweils flüssige wässrige und organische Phase die wässrige und die organische Phase jeweils zumindest teilweise ausschleust und die organische Phase, enthaltend den Acrylsäurebutylester und das Butanol, destillativ auftrennt.

Erfindungsgemäß wurde erkannt, dass mit dem erfindungsgemäßen Verfahren u.a. folgende Vorteile erzielt werden:
Durch den Einsatz von 3-Hydroxy-propionsäure kann ein nahezu Acetat-freier Acrylsäurebutylester hergestellt werden. Unter 'Acetat' ist hier n-Butyl- bzw. iso-Butylacetat [H₃C-C(=O)-OR] zu verstehen.
In den konventionellen Anlagen zur Herstellung von Acrylsäure (als Zwischenstufe für die anschließende Veresterung) muss die bei der Reaktion neben der gebildeten Acrylsäure ebenfalls entstehende Essigsäure aufwendig abgetrennt werden, um eine Acetatbildung bei der anschließenden Veresterung mit dem Alkohol zu vermeiden.
Weiterhin wird in einer besonderen Ausgestaltung durch ein verbessertes Regelkonzept (s.u.) eine nochmals höhere Ausbeute an Acrylsäurebutylester bei niedrigerem Energieverbrauch und nochmals verbesserter Qualität (insb. Reinheit) erzeugt.
Darüber hinaus werden in besonderen Ausgestaltungen weiter erhöhte Ausbeuten und/oder Produktreinheiten durch eine besondere Anfahrstrategie (s.u.) und/oder ein besonderes Stabilisierungskonzept (s.u.) erzielt.

In einer bevorzugten Verfahrenvariante handelt es sich bei der eingesetzten 3-Hydroxy-propionsäure um biobasierte 3-Hydroxy-propionsäure.
Unter "biobasierter 3-Hydroxy-propionsäure" ist eine solche zu verstehen, die ausgehend von nachwachsenden Rohstoffen hergestellt wurde.
Weiter bevorzugt ist, dass die biobasierte 3-Hydroxy-propionsäure durch Fermentation, insb. aus Glukose, Xylose, Arabinose, Sukrose, Fruktose, Cellulose, Glukose-Oligomere und/oder Glycerin durch Fermentation, besonders mit anschließender Reinigung, hergestellt wurde. Z.B. ist aus WO 2012/074818 A2 (Novozymes, Inc.) die Herstellung von biobasierter 3-Hydroxy-propionsäure (kurz: Bio-3-Hydroxy-propionsäure, noch kürzer: Bio-HPS) aus Zuckern wie Glukose durch Fermentation und anschließender Reinigung bekannt.
Die so erzeugte wässrige Bio-3-Hydroxy-propionsäure enthält beispielsweise neben Wasser im Wesentlichen folgende Bestandteile:
35 bis 70 Gew.-% 3-Hydroxy-propionsäure.
0 bis 20 Gew.-% oligomere 3-Hydroxy-propionsäure,
0 bis 10 Gew.-% Acrylsäure,
0 bis 1 Gew.-% oligomere Acrylsäure
0,01 bis 0,1 Gew.-% Glykolsäure,
0,01 bis 0,1 Gew.-% 2-Hydroxy-propionsäure,
0,005 bis 0,05 Gew.-% Ameisensäure,
0 bis 0,15 Gew.-%, besonders 0,0 bis 0,05 Gew.-%, z.B. 0,005 bis 0,10 Gew.-%, Essigsäure,
0,005 bis 0,05 Gew.-% Bernsteinsäure,
0,005 bis 0,05 Gew.-% Fumarsäure,
0,0001 bis 0,01 Gew.-% Formaldehyd,
0,0001 bis 0,01 Gew.-% Acetaldehyd,
0,0001 bis 0,01 Gew.-% Methanol und
0,0001 bis 0,01 Gew.-% Ethanol.

Bevorzugt beträgt das molare Einsatzverhältnis von Butanol R-OH zu 3-Hydroxy-propionsäure ≥ 1 und liegt weiterhin bevorzugt unterhalb von 5. Besonders vorteilhaft ist ein molares Einsatzverhältnis von Butanol R-OH zu 3-Hydroxy-propionsäure im Bereich von 1 : 1 bis 3 : 1. Ganz besonders bevorzugt ist ein molares Einsatzverhältnis im Bereich von 1,1 : 1 bis 1,8 : 1.

Die dehydratisierenden und gleichzeitig veresternden Bedingungen sind bevorzugt dadurch gekennzeichnet, dass eine katalytisch wirksame Menge einer Säure zugegen ist. Gemäß einer vorteilhaften Ausbildung der Erfindung beträgt der Gehalt an katalytisch wirksamer Säure im Reaktor, bezogen auf das darin enthaltene Reaktionsgemisch, 0,1 bis 20 Gew.-%, besonders 5 bis 15 Gew.-%, weiter besonders 7 bis 10 Gew.-%. Bevorzugte Säuren sind Mineralsäuren, wie Schwefelsäure, Phosphorsäure, sowie organische Sulfonsäure. Untern den organischen Sulfonsäuren sind Methansulfonsäure, Benzolsulfonsäure, Dodecylbenzolsulfonsäure und/oder p-Toluolsulfonsäure bevorzugt. Auch kann ein Gemisch aus organischer(organischen) Sulfonsäure(n) und Mineralsäure(n), z.B. Schwefelsäure, eingesetzt werden. Besonders bevorzugt ist es, als Veresterungs- und Dehydratisierungskatalysator(en) Schwefelsäure und/oder organische Sulfonsäure(n) einzusetzen.

Die Umsetzung der Reaktanden, also der Edukte 3-Hydroxy-propionsäure und Butanol R-OH, im Reaktor erfolgt bevorzugt bei einer Temperatur im Bereich von 80 bis 170 °C, besonders im Bereich von 100 bis 155 °C, weiter besonders im Bereich von 120 bis 140 °C.

Die Verweilzeit der Reaktanden, also der Edukte 3-Hydroxy-propionsäure und Butanol R-OH, im Reaktor beträgt bevorzugt 1 bis 20, weiter bevorzugt 2 bis 8, Stunden. Unter der Verweilzeit versteht man die Zeit, welche die Sumpfabzugsmenge (4) in dem Flüssigkeitsvolumen des Reaktionsbehälters (1) verweilt.

Im einfachsten Fall ist die Rektifikationskolonne (6) direkt auf den Reaktionsbehälter (1) (Reaktionsbehälter = Reaktor) aufgesetzt, wobei in der Regel die aus dem Reaktionsbehälter aufsteigenden Brüden (5) zu den in die Rektifikationskolonne geführten Rücklaufmengen (13; 15) im Gegenstrom geführt werden.
Die direkt aufgesetzte Kolonne bietet den Vorteil, die im Reaktor (1) entstehenden Brüden (5) unmittelbar ohne zusätzliche Rohrleitungen in die Rektifikationskolonne (6) und die in der Kolonne ablaufende Flüssigkeit (7) direkt in den Reaktor (1) zu befördern.
Es ist jedoch auch eine getrennte Anordnung von Reaktionsbehälter (1) und Rektifikationskolonne (6) möglich, mit entsprechenden Rohrleitungen zur Zuführung der Brüden in die Kolonne sowie zum Ablauf der durch die Rektifikationskolonne strömenden Flüssigkeit in den Reaktionsbehälter. Auch solch eine Ausführungsform mit indirekt aufgesetzter Kolonne ist vom Begriff 'Reaktor mit Rektifikationskolonne' umfasst.
Die Rektifikationskolonne (6) ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. Die Wirksamkeit derartiger Einbauten innerhalb der Rektifikationskolonne sollte bevorzugt wenigstens fünf theoretischen Stufen, z.B. im Bereich von 6 bis 40 theoretischen Stufen, entsprechen; besonders bevorzugt ist eine Trennleistung von 10 bis 30 theoretischen Stufen vorzusehen.

Im erfindungsgemäßen Verfahren liegt der Druck am Kopf der Rektifikationskolonne (6) bevorzugt im Bereich von 0,2 bis 5,0 bar, besonders im Bereich von 0,3 bis 3,0 bar, weiter besonders im Bereich von 0,5 bis 1,2 bar.

Bevorzugt erfolgt die Scheidung in eine wässrige und eine organische Phase mittels eines Phasenscheiders. In einem solchen Apparat können zwei nicht miteinander homogen mischbare Flüssigkeiten durch deren Dichteunterschied getrennt werden. Zumindest ein Teil der anfallenden wässrigen Phase, die neben Wasser auch noch Butanol R-OH und in Spuren ggf. weitere Komponenten enthält, wird ausgeschleust. Besonders bevorzugt werden 10 bis 80 Gew.-%, weiter besonders 20 bis 70 Gew.-%, der anfallenden wässrigen Phase ausgeschleust. Der Rest wird jeweils, bevorzugt in die Rektifikationskolonne (6), zurückgeführt.
Bevorzugt wird ein Teil der anfallenen organischen Phase ebenfalls, bevorzugt in die Rektifikationskolonne, zurückgeführt. Besonders werden 0 bis 80 Gew.-%, z.B. 1 bis 75 Gew.-%, weiter besonders 5 bis 50 Gew.-% der organischen Phase, bevorzugt in die Rektifikationskolonne, zurückgeführt. Der andere Teil wird ausgeschleust, und einer destillativen Auftrennung zugeführt.
Die destillative Auftrennung der ausgeschleusten organischen Phase enthaltend den Acrylsäurebutylester und das Butanol (R-OH) erfolgt bevorzugt derart, dass in einer nachgeschalteten Rektifikationskolonne das Butanol über Kopf abgetrennt wird (wie z.B. in EP 765 859 A1 (BASF AG) beschrieben). Vorzugsweise führt man das so abgetrennte Butanol in den Reaktor zurück (s.u.). Zweckmäßigerweise erfolgt die Rückführung kontinuierlich, mit oder ohne Zwischenbehälter.
Die resultierende Sumpfflüssigkeit dieser Rektifikationskolonne besteht im Wesentlichen aus dem Acrylsäurebutylester sowie geringen Mengen an Schwersiedern und eingesetztem Stabilisator (z.B. sog. Prozessstabilisator; z.B. insbesondere Phenothiazin (PTZ)).
In einer weiteren nachgeschalteten Rektifikationskolonne wird der Acrylsäurebutylester üblicherweise über Kopf abgetrennt. Bei der Kondensation wird bevorzugt ein Stabilisator (sog. Lagerstabilisator; z.B. insbesondere MeHQ) zugegeben. Die die schwerer siedenden Nebenprodukte enthaltende Sumpfflüssigkeit dieser Rektifikationskolonne wird zweckmäßigerweise bevorzugt in den Reaktor zurückgeführt, vorzugsweise kontinuierlich, mit oder ohne Zwischenbehälter.

Eine besondere Ausführungsform (ebenfalls beschrieben in EP 765 859 A1 (BASF AG)) besteht darin, dass der Acrylsäurebutylester aus der Rektifikationskolonne zur Rückgewinnung von Butanol durch einen Seitenabzug nach Abscheidung eventuell mitgerissener Flüssigkeitströpfchen entnommen und zum Reinester kondensiert wird. Bei der Kondensation wird diesem ein Stabilisator (der sog. Lagerstabilisator; z.B. insbesondere p-Methoxy-phenol (MeHQ)) zugegeben. In diesem Ausführungsfall wird der Sumpfaustrag der Butanol-Rektifikationskolonne (in der Hauptsache bestehend aus Acrylsäurebutylester) vorzugsweise zurück in den Reaktor geführt.

Das nach der Auftrennung erhaltene Butanol wird besonders vorteilhaft zumindest teilweise zur Umsetzung in den Reaktor zurückgeführt. Bevorzugt werden 5 bis 100 Gew.-%, weiter bevorzugt 80 bis 100 Gew.-%, des Alkohols zurückgeführt.

Das erfindungsgemäße Verfahren wird besonders bevorzugt zur Herstellung von Acrylsäure-n-butylester, wobei man wässrige, besonders biobasierte, 3-Hydroxy-propionsäure in Gegenwart des Alkohols n-Butanol umsetzt, eingesetzt.

Mit dem erfindungsgemäßen Verfahren kann Acrylsäure-n-butylester mit insbesondere einer Reinheit von ≥ 99,0 Gew.-%, weiter besonders ≥ 99,5 Gew.-%, und einem Gehalt an n-Butylacetat von ≤ 1000 ppm, weiter besonders von ≤ 100 ppm, hergestellt werden. Insbesondere beträgt der Gehalt an Acrylsäure < 100 ppm, z.B. 5 bis 80 ppm.

Mit dem erfindungsgemäßen Verfahren kann Acrylsäure-iso-butylester mit insbesondere einer Reinheit von ≥ 99,0 Gew.-%, weiter besonders ≥ 99,5 Gew.-%, und einem Gehalt an iso-Butylacetat von ≤ 1000 ppm, weiter besonders von ≤ 100 ppm, hergestellt werden. Insbesondere beträgt der Gehalt an Acrylsäure < 100 ppm, z.B. 5 bis 80 ppm.
Im erfindungsgemäßen Verfahren wird der gebildete Acrylsäurebutylester bevorzugt durch geeignete Polymerisationsinhibitoren stabilisiert, um eine ungewünschte Polymerisation zu vermeiden. D.h., das erfindungsgemäße Verfahren wird bevorzugt in Gegenwart wirksamer Mengen eines Stabilisators oder mehrerer Stabilisatoren durchgeführt. Als Stabilisatoren eignen sich prinzipiell alle Polymerisationsinhibitoren, die zur Stabilisierung von Acrylsäure und Acrylsäureestern in z.B. DE 10 2005 053 982 A1 (BASF AG) und DE 102 58 329 A1 (BASF AG) empfohlen werden.
Geeignete Stabilisatoren können beispielsweise N-Oxide (Nitroxyl- oder N-Oxyl-Radikale, also Verbindungen, die wenigstens eine >N-O-Gruppe aufweisen), wie z.B. 4-Hydroxy-2,2,6,6-tetramethylpiperidin-N-oxyl (4HT) oder 4-Oxo-2,2,6,6-tetramethylpiperidin-N-oxyl, Phenole und Naphthole, wie p-Methoxyphenol, p-Aminophenol, p-Nitrosophenol, 2-tert.-Butylphenol, 4-tert.-Butylphenol, 2,4-Di-tert.-butylphenol, 2-Methyl-4-tert.-butylphenol, 2,6-tert.-Butyl-4-methylphenol oder 4-tert.-Butyl-2,6-dimethylphenol, Chinone, wie z.B. Hydrochinon oder Hydrochinonmonomethylether, aromatische Amine, wie z.B. N,N-Diphenylamin, Phenylendiamine, wie z.B. N,N'-Dialkyl-p-phenylendiamin, wobei die Alkylreste gleich oder verschieden sein können und jeweils unabhängig voneinander 1 bis 4 Kohlenstoffatome aufweisen und geradkettig oder verzweigt sein können, wie z.B. N,N'-Dimethyl-p-phenylendiamin oder N,N'-Diethyl-p-phenylendiamin, Hydroxylamine, wie z.B. N,N-Diethylhydroxylamin, Imine, wie z.B. Methylethylimin oder Methylenviolett, Sulfonamide, wie z.B. N-Methyl-4-toluolsulfonamid oder N-tert.-Butyl-4-toluolsulfonamid, Oxime, wie Aldoxime, Ketoxime oder Amidoxime, wie z.B. Diethylketoxim, Methylethylketoxim oder Salicyladoxim, phosphorhaltige Verbindungen, wie z.B. Triphenylphosphin, Triphenylphosphit oder Triethylphosphit, schwefelhaltige Verbindungen wie z.B. Diphenylsulfid oder Phenothiazin, Metallsalze, wie z.B. Cer(III)acetat oder Cer(III)ethylhexanoat, aber auch verschiedene Kupfersalze etwa Cu(II)dialkyldithiocarbamate wie z.B. das Cu(II)dibutyldithiocarbamat und auch Cu(II)oxinat (Oxin = 4-Hydroxychinolin), zudem Mangansalze wie z.B das Mn(II)diacetat oder Gemische davon, sein.
Bevorzugt erfolgt die Stabilisierung mit Phenothiazin (PTZ), p-Methoxyphenol (MeHQ), Hydrochinon, Hydrochinonmonomethylether, 4-Hydroxy-2,2,6,6-tetramethylpiperidin-N-oxyl, 4-Oxo-2,2,6,6-tetramethylpiperidin-N-oxyl, 2,6-tert.-Butyl-4-methylphenol oder Gemischen davon. Ganz besonders bevorzugt wird Phenothiazin (PTZ) und/oder p-Methoxyphenol (MeHQ) und/oder 4-Hydroxy-2,2,6,6-tetramethylpiperidin-N-oxyl (4HT) als Polymerisationsinhibitor verwendet.
Auch wenn die Inhibitoren als Reinsubstanz zugegeben werden können, ist es von Vorteil, den Inhibitor gelöst in einem Lösungsmittel als einfach und reproduzierbar zu dosierende Lösung zuzugeben, wobei grundsätzlich auch Inhibitormischungen in einer einzelnen Lösung möglich sind. Bevorzugt wird eine im Acrylatsyntheseverfahren bzw. dem Stoffgemisch in der Kolonne bereits vorhandene Flüssigkeit als Lösungsmittel verwendet. Besonders bevorzugt für die Wahl als Lösungsmittel ist das Acrylatprodukt (also der Acrylsäurebutylester) selbst, Wasser oder einer der Syntheseeinsatzstoffe für das Acrylat (z.B. das Butanol R-OH).

Das erfindungsgemäße Verfahren wird vorteilhaft mit besonderen Maßnahmen zur Kontrolle bestimmter Parameter durchgeführt. Diese Prozesskontrolle erfolgt bevorzugt wie folgt:
Für die Produktion von spezifikationsgerechtem Acrylsäurebutylester, also einem Produkt mit hoher Reinheit, ist die Abtrennung von Acrylsäure vom Acrylsäurebutylester in der Rektifikationskolonne (6) von entscheidender Bedeutung.
Dabei hat sich als vorteilhaft erwiesen, ein definiertes Verhältnis zwischen dem organischen (13) und dem wässrigen Rücklauf (15) einzustellen. Dieses Rücklaufverhältnis der Ströme 13 zu 15 liegt bevorzugt im Bereich von 0,1 - 1,0.
Weiterhin sollte das für den Umsatz ausschlaggebende Reaktionsvolumen im Sumpf (1) der Kolonne (oder eigenständigem Reaktionsbehälter bei indirekt aufgesetzter Kolonne) bevorzugt konstant oder nahezu konstant (+/- 10 Vol.%) gehalten werden. Dies lässt sich zu einem dadurch erreichen, dass bei konstantem oder nahezu konstantem (+/-10 Vol.%) Flüssigkeitsstand im Reaktionsbehälter ein konstanter oder nahezu konstanter (+/- 10 Vol.%) Flüssigkeitsstrom (4) aus dem Reaktionsvolumen (1) ausgeschleust wird. Außerdem sollte bevorzugt die Sumpfabzugsmenge (4) in einem bestimmten Verhältnis zum Zulauf (3) stehen, bevorzugt in einem Verhältnis der Ströme 4 zu 3 im Bereich von 0,01 - 0,30.
Eine zweite Maßnahme ist eine Qualitätskontrolle bezüglich des Acrylsäuregehaltes im organischen Destillat (14). Da das Flüssigkeitsvolumen in dem Reaktionsraum (1) stark auf die wässrige Rücklaufmenge (15) reagiert, wird der Flüssigkeitsstand im Reaktor (1) bevorzugt mittels der Rücklaufmenge (= Rückführmenge) der wässrigen Phase (15) geregelt. Der wässrige Rücklauf gewährleistet, dass die Schwersieder n- bzw. iso-Butylacrylat und das entsprechende Butanol (R-OH) aufgrund der Bildung eines Leichtsiederazeotrops abdestilliert werden können. Der organische Rücklauf gewährleistet, dass die Konzentration der im Reaktor (1) entstandenen Acrylsäure unter der Konzentration von insbesondere 100 ppm bleibt.
Durch die Steuerung der Menge des organischen Rücklaufs (13) können mehrere Effekte kombiniert werden: Reinigen durch Destillieren, Erhöhung der Verweilzeit im Reaktionsraum, Erhöhung der Konzentration an Butanol (R-OH) im Reaktionsraum (1).
Diese Regelstrategie führt zu einem besonders stabilen Betrieb im Reaktionsbehälter (1) und in der Rektifikationskolonne (6).

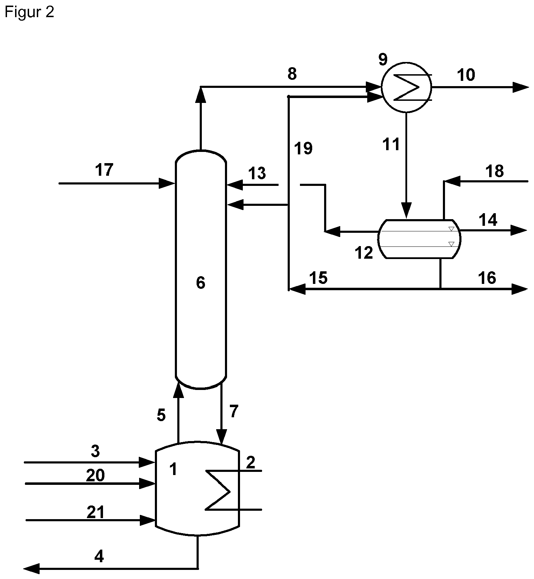

In einer bevorzugten Ausführungsform (vgl. Figur 2) ist im erfindungsgemäßen Verfahren in der Rektifikationskolonne (6) mindestens ein Stabilisator (Stabilisator 1) zugegen, der sich in wirksamen Anteilen sowohl in der wässrigen als auch in der organischen Phase löst. Besonders wird ein solcher Stabilisator, wie insbesondere 4HT, oberhalb der obersten theoretischen Trennstufe der Kolonne (6) zugegeben (17). Dadurch ist die gesamte Rektifikationskolonne (6) mit dem Stabilisator stabilisiert.
Weiterhin (vgl. Figur 2) wird im erfindungsgemäßen Verfahren bevorzugt mindestens ein Stabilisator, der sich in wirksamen Anteilen sowohl in der wässrigen als auch in der organischen Phase löst, in den Phasenscheider (12), der das Kondensat (11) auffängt, (18) und/oder in die Leitung eines Quenchkreislaufs (19) und/oder am Kopf des Kondensators (9) zugegeben. Dieser Stabilisator gleicht bevorzugt dem Stabilisator 1 (insb. 4HT). Der bevorzugt eingerichtete Quenchkreislauf (d.h. der flüssige Rückführstrom eines Teils des Kondensats, z.B. 10 bis 50 Gewichtshundertstel des Kondensats, in den Kondensator (9)) hat die Funktion, dass die natürlicherweise stabilisatorfreien Brüden (8) beim Kondensieren im Kondensator (9) besonders ausreichend stabilisiert sind. In der jeweiligen Phase in Lösung befindliche wirksame Stabilisatormengen liegen insgesamt besonders bei ≥ 10 Gew.-ppm, z.B. im Bereich von 10 bis 1000 Gew.-ppm. Wenn sich ein betreffend eingesetzter Stabilisator in der jeweiligen flüssigen Phase nicht vollständig löst, ist er entsprechend suspendiert zugegen. Bei Vorhandensein eines Stabilisators als Suspension in der oder den flüssigen Phasen kann dieser ä priori kaum oder nicht wirksame partikuläre Stabilisatoranteil durch seine Wirkung als Stabilisator-Depot Vorteile bieten, da z.B. im Falle des chemischen Abbaus von gelöstem Stabilisator, wodurch dessen Wirksamkeit verschlechtert wird, weiterer, dann frisch wirksamer Stabilisator aus dem suspendierten Anteil zusätzlich in Lösung geht, was bei geeignet innigem Kontakt der flüssigen Phasen auch phasen-übergreifend eintreten kann und über die Grössenverteilung der Partikel beeinflussbar ist. Die Stabilisatoren können jeweils insbesondere als Lösung in einem geeigneten Lösungsmittel, besonders wie oben aufgeführt, z.B. dem im Verfahren verwendeten Alkohol, Wasser, dem entsprechenden Acrylsäurebutylester, z.B. jeweils als 1 - 5 Gew.-%ige Lösung, eingesetzt werden (z.B. über die Leitungen 17 bzw. 18, Figur 2).
In den Reaktionsbehälter (1) wird vorteilhaft ein Stabilisator 2 (20), insbesondere PTZ, der für höhere Temperaturen geeignet ist, und ein Stabilisator 3 (20) (insbesondere MeHQ) der aufgrund seines höheren Dampfdruckes auch noch den Übergangsbereich zwischen Reaktionsraum und Kolonnenunterteil stabilisiert, zugegeben.
Die Stabilisatoren 2 und 3 können jeweils insbesondere als Lösung in einem geeigneten Lösungsmittel, besonders wie oben aufgeführt, z.B. dem im Verfahren entsprechend gebildeten Acrylsäurebutylester oder in den verwendeten Edukten 3HPA oder Butanol (R-OH) eingesetzt werden (z.B. über die Leitung 20, Figur 2).
Vorteilhaft wird im erfindungsgemäßen Verfahren zusätzlich ein Sauerstoff-haltiges Gas zur Polymerisationsinhibierung eingesetzt. Besonders geeignet sind hierzu Luft/StickstoffGemische, z.B. mit einem Sauerstoffgehalt von 4 bis 9 Vol.-%.
Wird ein Sauerstoff-haltiges Gas zur Polymerisationsinhibierung eingesetzt, so wird dies vorzugsweise am unteren Ende des Verdampfers (2) oder am unteren Ende des Reaktionsraumes (1) zugeführt (vgl. 21 in Figur 2).

Das Anfahren des erfindungsgemäßen Verfahrens umfassend die Umsetzung im Reaktionsbehälter (1) und die Destillation in der Kolonne (6) kann problembehaftet sein, da insbesondere Änderungen der Rücklaufmengen der beiden Ströme 13 und 15 sich stark unterschiedlich auf das Gesamtsystem auswirken.
Änderungen der Menge des wässrigen Rücklaufs (15) wirken sich relativ schnell auf die gebildete Dampfmenge und Änderungen der Menge des organischen Rücklaufs (13) wirken sich relativ langsam auf die Acrylsäurekonzentration im Kopf der Kolonne aus. Aber beide Rücklaufmengen sind nicht unabhängig voneinander. Erfindungsgemäß wurde erkannt: Wenn die genauen Rücklaufmengen nicht gut aufeinander abgestimmt sind, dann kann die Verdampfung zum Erliegen kommen, oder die Kolonne (6) kommt aufgrund zu hoher Dampfmenge zum Fluten. Es ist dann sehr schwierig, das System wieder in den normalen Betriebszustand zu bekommen.
Vorteilhaft wird daher zum Anfahren der Behälter 1 (der Reaktor) zunächst mit entsprechender Menge eines entsprechenden Reaktionsgemisches enthaltend den Acrylsäurebutylester, insbesondere Sumpfprodukt aus einer vorhergehenden Produktionskampagne, oder des entsprechenden Acrylsäurebutylesters befüllt. Dann wird der Sumpf auf Betriebstemperatur (Umsetzungstemperatur) erhitzt und die Zuläufe aus 3HPA, Butanol und Katalysator in Betrieb genommen.

Alle Druckangaben beziehen sich auf den Absolutdruck.
Alle ppm-Angaben beziehen sich auf das Gewicht.

### Beispiele

### Beispiel 1a

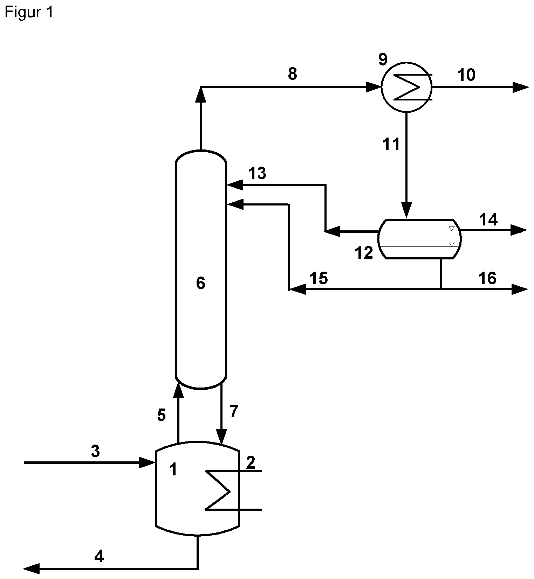

Figur 1 zeigt ein Ausführungsbeispiel für das erfindungsgemäße Verfahren bis zur Gewinnung des Gemisches enthaltend den Acrylsäurealkylester und den Alkohol.

Die Figur 1 zeigt einen Reaktionsbehälter 1 (= Reaktor), eine Rektifikationskolonne 6 mit trennwirksamen Einbauten. Über eine Zuführungsleitung 3 wird ein Gemisch aus wässriger 3-Hydroxy-propionsäure (3HPA), und Butanol (R-OH) sowie p-Toluolsulfonsäure als Veresterungskatalysator zugeführt. Das gasförmige Reaktionsprodukt wird vom Kopf des Reaktors 1 über eine Leitung 5 einer Rektifikationskolonne 6 zugeführt. Die am unteren Teil des Reaktors 1 vorgesehene Leitung 4 dient der Entsorgung verbliebener Rückstände.
Das aus der Reaktionszone kontinuierlich in die Rektifikationskolonne 6 strömende Dampfgemisch wird dort rektifiziert, und das vom Kopf dieser Rektifikationskolonne 6 über die Leitung 8 abströmend, den zu bildenden Zielester enthaltende wässrige Azeotrop, das überwiegend aus Acrylat, Alkohol und Wasser besteht, wird einem Kondensator 9, der gegebenenfalls durch einen Nachkühler ergänzt wird, zugeführt und darin teilweise kondensiert. Der nicht kondensierte Anteil aus dem Kondensator 9 enthält die niedersiedenden Verunreinigungen und wird dampfförmig als Strom 10 abgezogen.
Das Kondensat strömt über eine Leitung 11 in einen Flüssig-Flüssig-Phasenscheider 12. Dort scheidet sich das Azeotrop in eine wässrige und in eine organische Phase. Die wässrige Phase, die hauptsächlich aus Wasser und etwas Alkohol und Acrylat besteht, wird teilweise über die Leitung 15 dem Kopf der Rektifikationskolonne 6 zugeführt, um die rektifikatorische Trennung wie auch die Azeotropbildung der dort aufsteigenden Dämpfe zu bewirken. Ein weiterer Teil wird über Leitung 16 zur Abstrippung von Alkohol entnommen. Aus dem Abscheider 12 wird organischer Rücklauf über die Leitung 13 in den Kopf der Rektifikationskolonne 6 eingeführt, um die Acrylsäure zurückzudrängen.

Am unteren Kolonnenende wird die Flüssigkeit 7 in einem Verdampfer 2 teilweise verdampft und über die Rohrleitung 5 in die Kolonne zurückgeführt. Ein Teilstrom 4, der die höhersiedenden Verunreinigungen enthält, wird abgezogen. Der Verdampfer 2 kann als beheizbarer Behälter, als Naturumlaufverdampfer oder als Zwangsumlaufverdampfer ausgebildet sein, im letzteren Fall ist zusätzlich eine Umlaufpumpe für den Flüssigkeitsstrom 4 erforderlich. Besonders vorteilhaft hinsichtlich der Vermeidung unerwünschter Polymerisationsreaktionen ist ein Zwangsumlaufentspannungsverdampfer einzusetzen, da mit dieser Bauart keine Verdampfung an den beheizten Flächen stattfindet.

### Beispiel 1b

Die Fahrweise wird anhand der Daten aus einer thermodynamischen Simulation einer Gesamtanlage zur Herstellung von Butylacrylat dargestellt.
Die thermodynamische Simulation des Prozesses wurde mit der Software Aspen Plus® (kurz: Aspen) durchgeführt. Aspen ist eine umfangreiche Simulationssoftware, die zur Modellierung, Simulation und Optimierung chemischer Verfahren und Anlagen in der Industrie eingesetzt wird. Aspen verfügt über umfangreiche Modell-Datenbanken zur Modellierung der Basisoperationen sowie über Stoffdatenbanken für die Stoffeigenschaften vieler verschiedener Substanzen. Die Eigenschaften von Mischungen werden von Aspen mit Hilfe von unterschiedlichen thermodynamischen Modellen aus den Stoffdaten der reinen Substanzen berechnet.
Die thermodynamische Simulation der Gesamtanlage führte zu folgenden Ergebnissen:
Über eine Zuführungsleitung 3 wird folgendes Gemisch dem Reaktor 1 zugeführt:

| | |
|---|---|
| wässrige 3-Hydroxy-propionsäure (3HPA): | 1000 g/h |
| n-Butanol: | 953 g/h |
| p-Toluolsulfonsäure: | 4 g/h |

Über eine Zuführungsleitung 20 wird folgendes Gemisch dem Reaktor 1 zugeführt:

| | |
|---|---|
| n-Butanol: | 9,8 g/h |
| MeHQ: | 0,1 g/h |
| PTZ: | 0,1 g/h |

Über eine Zuführungsleitung 21 wird ein Sauerstoff-haltiges Gas dem Reaktor 1 zugeführt:

| | |
|---|---|
| Stickstoff: | 9,5 l/h |
| Sauerstoff: | 0,5 l/h |

Die wässrige 3-Hydroxy-propionsäure hat folgende Zusammensetzung:

| | |
|---|---|
| Wasser: | 20 Gew.-% |
| 3-Hydroxy-propionsäure: | 80 Gew.-% |
| Essigsäure: | < 0,01 Gew.-% |

Über eine Zuführungsleitung 18 wird folgendes Gemisch dem Phasenscheider 12 zugeführt:

| | |
|---|---|
| Wasser: | 9,9 g/h |
| 4HT: | 0,1 g/h |

Die Umsetzung wird bei einer Temperatur von 150 °C, einem Druck von 1 bar und einer Verweilzeit von 2 h durchgeführt.
Aus dem Flüssig-Flüssig-Phasenscheider 12 werden 217 g/h der organischen Phase als Rücklauf über die Leitung 13 zur Kolonne 6 zurückgefahren und 1444 g/h über die Leitung 14 als organisches Destillat abgezogen.
Die organische Phase hat folgende Zusammensetzung:

| | |
|---|---|
| Wasser: | 7 Gew.-% |
| n-Butanol: | 23 Gew.-% |
| n-Butylacrylat: | 70 Gew.-% |
| Acrylsäure: | < 0,01 Gew.-% |
| n-Butylacetat: | < 0,01 Gew.-% |
| 4HT: | < 0,01 Gew.-% |

Aus dem Flüssig-Flüssig-Phasenscheider 12 werden 425 g/h der wässrigen Phase als Rücklauf über die Leitung 15 zur Kolonne 6 zurückgefahren und 425 g/h über die Leitung 16 als wässriges Destillat abgezogen.
Die wässrige Phase hat folgende Zusammensetzung:

| | |
|---|---|
| Wasser: | 95 Gew.-% |
| n-Butanol: | 3 Gew.-% |
| n-Butylacrylat: | 2 Gew.-% |
| Acrylsäure: | < 0,01 Gew.-% |
| n-Butylacetat: | < 0,01 Gew.-% |
| 4HT: | < 0,01 Gew.-% |

Am unteren Teil des Reaktors wird über die Leitung 4 108 g/h als flüssiger Rückstand abgezogen.
Der Rückstand hat folgende Zusammensetzung:

| | |
|---|---|
| Wasser: | 1 Gew.-% |
| n-Butanol: | 3 Gew.-% |
| n-Butylacrylat: | 16 Gew.-% |
| Acrylsäure: | 3 Gew.-% |
| n-Butylacetat: | < 0,01 Gew.-% |
| Schwersieder: | 77 Gew.-% |
| MeHQ: | < 0,1 Gew.-% |
| PTZ: | < 0,1 Gew.-% |
| 4HT: | < 0,01 Gew.-% |

Die organische Phase kann dann nach den bekannten Verfahrensschritten (insbesondere gemäß EP 765 859 A1 (BASF AG), vgl. oben Seite 8) zum Reinprodukt n-Butylacrylat aufgearbeitet werden.
Das Reinprodukt hat folgende Zusammensetzung:

| | |
|---|---|
| Wasser: | < 0,01 Gew.-% |
| n-Butanol: | < 0,01 Gew.-% |
| n-Butylacrylat: | > 99,5 Gew.-% |
| Acrylsäure: | < 0,01 Gew.-% |
| n-Butylacetat: | < 0,01 Gew.-% |

Das Beispiel belegt beispielhaft die vorteilhaften Eigenschaften bezüglich des geringen n-Butylacetatgehaltes im Reinprodukt.

## Patentansprüche

1. Verfahren zur kontinuierlichen Herstellung eines Acrylsäurebutylesters H₂C=CH-C(=O)OR, mit R = n-Butyl oder iso-Butyl, **dadurch gekennzeichnet, dass** man wässrige 3-Hydroxy-propionsäure unter dehydratisierenden und veresternden Bedingungen in Gegenwart des entsprechenden Butanols R-OH in einem Reaktor mit Rektifikationskolonne umsetzt und gebildeten Acrylsäurebutylester, unumgesetztes Butanol sowie eingesetztes und gebildetes Wasser als ternäres Azeotrop über Kopf abdestilliert, nach Scheidung in eine jeweils flüssige wässrige und organische Phase die wässrige und die organische Phase jeweils zumindest teilweise ausschleust und die organische Phase, enthaltend den Acrylsäurebutylester und das Butanol, destillativ auftrennt.

2. Verfahren nach dem vorhergehenden Anspruch, **dadurch gekennzeichnet, dass** es sich bei der eingesetzten 3-Hydroxy-propionsäure um biobasierte 3-Hydroxy-propionsäure handelt.

3. Verfahren nach dem vorhergehenden Anspruch, **dadurch gekennzeichnet, dass** die biobasierte 3-Hydroxy-propionsäure durch Fermentation hergestellt wurde.

4. Verfahren nach einem der vorhergehenden Ansprüche, **dadurch gekennzeichnet, dass** das molare Einsatzverhältnis von Butanol R-OH zu 3-Hydroxy-propionsäure ≥ 1 beträgt.

5. Verfahren nach einem der vorhergehenden Ansprüche, wobei die dehydratisierenden und veresternden Bedingungen **dadurch gekennzeichnet sind, dass** eine katalytisch wirksame Menge einer Säure zugegen ist.

6. Verfahren nach dem vorhergehenden Anspruch, **dadurch gekennzeichnet, dass** es sich bei der Säure um Schwefelsäure und/oder eine organische Sulfonsäure handelt.

7. Verfahren nach einem der vorhergehenden Ansprüche, **dadurch gekennzeichnet, dass** die Umsetzung im Reaktor bei einer Temperatur im Bereich von 80 bis 170 °C erfolgt.

8. Verfahren nach einem der vorhergehenden Ansprüche, **dadurch gekennzeichnet, dass** die Verweilzeit der Reaktanden im Reaktor im Bereich von 1 bis 20 Stunden liegt.

9. Verfahren nach einem der vorhergehenden Ansprüche, **dadurch gekennzeichnet, dass** die Rektifikationskolonne ≥ 5 theoretische Stufen aufweist.

10. Verfahren nach einem der vorhergehenden Ansprüche, **dadurch gekennzeichnet, dass** der Druck am Kopf der Rektifikationskolonne im Bereich von 0,2 bis 5,0 bar (absolut) liegt.

11. Verfahren nach einem der vorhergehenden Ansprüche, **dadurch gekennzeichnet, dass** die destillative Auftrennung der organischen Phase, enthaltend den Acrylsäurebutylester und das Butanol, derart erfolgt, dass in einer Rektifikationskolonne das Butanol abdestilliert und aus der resultierenden Sumpfflüssigkeit in einer weiteren Rektifikationskolonne der Acrylsäurebutylester abdestilliert wird.

12. Verfahren nach einem der vorhergehenden Ansprüche, **dadurch gekennzeichnet, dass** das nach der Auftrennung erhaltene Butanol zumindest teilweise zur Umsetzung in den Reaktor zurückgeführt wird.

13. Verfahren nach einem der vorhergehenden Ansprüche zur Herstellung von Acrylsäure-n-butylester, wobei man 3-Hydroxy-propionsäure in Gegenwart von n-Butanol umsetzt.

14. Verfahren nach einem der Ansprüche 1 bis 12 zur Herstellung von Acrylsäure-iso-butylester, wobei man 3-Hydroxy-propionsäure in Gegenwart von iso-Butanol umsetzt.

15. Verfahren nach Anspruch 13 zur Herstellung von Acrylsäure-n-butylester mit einer Reinheit ≥ 99,0 Gew.-% und einem Gehalt an n-Butylacetat von ≤ 1000 Gew.-ppm.

16. Verfahren nach Anspruch 14 zur Herstellung von Acrylsäure-iso-butylester mit einer Reinheit ≥ 99,0 Gew.-% und einem Gehalt an iso-Butylacetat von ≤ 1000 Gew.-ppm.

17. Verfahren nach einem der vorhergehenden Ansprüche, **dadurch gekennzeichnet, dass** man die Umsetzung in Gegenwart wirksamer Mengen eines Stabilisators oder mehrerer Stabilisatoren durchführt.

18. Verfahren nach einem der vorhergehenden Ansprüche, **dadurch gekennzeichnet, dass** der Flüssigkeitsstand im Reaktor mittels der Rücklaufmenge der wässrigen Phase geregelt wird.

19. Verfahren nach einem der vorhergehenden Ansprüche, **dadurch gekennzeichnet, dass** zum Anfahren des Verfahrens der entsprechende Acrylsäurebutylester oder ein entsprechendes Reaktionsgemisch enthaltend den Acrylsäurebutylester im Reaktor vorgelegt wird.

20. Verfahren nach einem der vorhergehenden Ansprüche, **dadurch gekennzeichnet, dass** sowohl in der Rektifikationskolonne als auch in der nach der Abdestillation resultierenden organischen Phase jeweils mindestens ein Stabilisator zugegen ist, der sich in wirksamen Anteilen sowohl in der wässrigen als auch in der organischen Phase löst.

## Claims

1. A process for continuously preparing a butyl acrylate H₂C=CH-C(=O)OR, with R = n-butyl or isobutyl, which comprises converting aqueous 3-hydroxypropionic acid under dehydrating and esterifying conditions in the presence of the corresponding butanol R-OH in a reactor with a rectification column and distilling off butyl acrylate formed, unconverted butanol and water used and formed overhead as a ternary azeotrope, after separation into a liquid aqueous phase and liquid organic phase at least partly discharging each of the aqueous and organic phases, and distillatively separating the organic phase comprising the butyl acrylate and the butanol.

2. The process according to the preceding claim, wherein the 3-hydroxypropionic acid used is biobased 3-hydroxypropionic acid.

3. The process according to the preceding claim, wherein the biobased 3-hydroxypropionic acid has been prepared by fermentation.

4. The process according to any of the preceding claims, wherein the molar use ratio of butanol R-OH to 3-hydroxypropionic acid is ≥ 1.

5. The process according to any of the preceding claims, wherein the dehydrating and esterifying conditions have the characteristic feature that a catalytically active amount of an acid is present.

6. The process according to the preceding claim, wherein the acid is sulfuric acid and/or an organic sulfonic acid.

7. The process according to any of the preceding claims, wherein the conversion in the reactor is effected at a temperature in the range from 80 to 170°C.

8. The process according to any of the preceding claims, wherein the residence time of the reactants in the reactor is in the range from 1 to 20 hours.

9. The process according to any of the preceding claims, wherein the rectification column has ≥ 5 theoretical plates.

10. The process according to any of the preceding claims, wherein the pressure at the top of the rectification column is in the range from 0.2 to 5.0 bar (absolute).

11. The process according to any of the preceding claims, wherein the distillative separation of the organic phase comprising the butyl acrylate and the butanol is effected in such a way that the butanol is distilled off in a rectification column and the butyl acrylate is distilled out of the resulting bottoms liquid in a further rectification column.

12. The process according to any of the preceding claims, wherein the butanol obtained after the separation is at least partly recycled to the conversion in the reactor.

13. The process according to any of the preceding claims for preparation of n-butyl acrylate, wherein 3-hydroxypropionic acid is converted in the presence of n-butanol.

14. The process according to any of claims 1 to 12 for preparation of isobutyl acrylate, wherein 3-hydroxypropionic acid is converted in the presence of isobutanol.

15. The process according to claim 13 for preparation of n-butyl acrylate having a purity of ≥ 99.0% by weight and an n-butyl acetate content of ≤ 1000 ppm by weight.

16. The process according to claim 14 for preparation of isobutyl acrylate having a purity of ≥ 99.0% by weight and an isobutyl acetate content of ≤ 1000 ppm by weight.

17. The process according to any of the preceding claims, wherein the conversion is conducted in the presence of effective amounts of one stabilizer or a plurality of stabilizers.

18. The process according to any of the preceding claims, wherein the liquid level in the reactor is under closed-loop control via the reflux volume of the aqueous phase.

19. The process according to any of the preceding claims, wherein the process is started up by initially charging the reactor with the corresponding butyl acrylate or a corresponding reaction mixture comprising the butyl acrylate.

20. The process according to any of the preceding claims, wherein at least one stabilizer that dissolves in effective proportions both in the aqueous phase and in the organic phase is present both in the rectification column and in the organic phase that results after the distillative removal.

## Revendications

1. Procédé pour la préparation en continu d'un ester de butyle d'acide acrylique H₂C=CH-C(=O)OR, avec R = n-butyle ou iso-butyle, **caractérisé en ce qu'**on transforme de l'acide 3-hydroxy-propionique aqueux dans des conditions de déshydratation et d'estérification en présence du butanol correspondant R-OH dans un réacteur doté d'une colonne de rectification et on élimine par distillation par la tête l'ester de butyle d'acide acrylique formé, le butanol n'ayant pas réagi ainsi que l'eau utilisée et formée en tant qu'azéotrope ternaire, on évacue par éclusage à chaque fois au moins partiellement la phase aqueuse et la phase organique, après séparation en une phase aqueuse et une phase organique à chaque fois liquide, et on sépare par distillation la phase organique, contenant l'ester de butyle d'acide acrylique et le butanol.

2. Procédé selon la revendication précédente, **caractérisé en ce que** l'acide 3-hydroxy-propionique utilisé est de l'acide 3-hydroxy-propionique à base biologique.

3. Procédé selon la revendication précédente, **caractérisé en ce que** l'acide 3-hydroxy-propionique à base biologique a été préparé par fermentation.

4. Procédé selon l'une quelconque des revendications précédentes, **caractérisé en ce que** le rapport molaire de départ de butanol R-OH sur acide 3-hydroxy-propionique est ≥ 1.

5. Procédé selon l'une quelconque des revendications précédentes, les conditions de déshydratation et d'estérification étant **caractérisées en ce qu'**une quantité catalytique efficace d'un acide est présente.

6. Procédé selon la revendication précédente, **caractérisé en ce que** l'acide est l'acide sulfurique et/ou un acide sulfonique organique.

7. Procédé selon l'une quelconque des revendications précédentes, **caractérisé en ce que** la transformation est réalisée dans le réacteur à une température dans la plage de 80 à 170 °C.

8. Procédé selon l'une quelconque des revendications précédentes, **caractérisé en ce que** le temps de séjour des réactants dans le réacteur se situe dans la plage de 1 à 20 heures.

9. Procédé selon l'une quelconque des revendications précédentes, **caractérisé en ce que** la colonne de rectification présente ≥ 5 plateaux théoriques.

10. Procédé selon l'une quelconque des revendications précédentes, **caractérisé en ce que** la pression à la tête de la colonne de rectification se situe dans la plage de 0,2 à 5,0 bars (absolu).

11. Procédé selon l'une quelconque des revendications précédentes, **caractérisé en ce que** la séparation par distillation de la phase organique, contenant l'ester de butyle d'acide acrylique et le butanol, est réalisée de telle manière que le butanol est éliminé par distillation dans une colonne de rectification et l'ester de butyle d'acide acrylique est éliminé par distillation du liquide de fond résultant dans une colonne de rectification supplémentaire.

12. Procédé selon l'une quelconque des revendications précédentes, **caractérisé en ce que** le butanol obtenu après la séparation est au moins partiellement recyclé pour la transformation dans le réacteur.

13. Procédé selon l'une quelconque des revendications précédentes pour la préparation d'ester de n-butyle d'acide acrylique, dans lequel on transforme l'acide 3-hydroxy-propionique en présence de n-butanol.

14. Procédé selon l'une quelconque des revendications 1 à 12 pour la préparation d'ester d'iso-butyle d'acide acrylique, dans lequel on transforme l'acide 3-hydroxy-propionique en présence d'iso-butanol.

15. Procédé selon la revendication 13 pour la préparation d'ester de n-butyle d'acide acrylique doté d'une pureté ≥ 99,0 % en poids et d'une teneur en acétate de n-butyle de ≤ 1 000 ppm en poids.

16. Procédé selon la revendication 14 pour la préparation d'ester d'iso-butyle d'acide acrylique doté d'une pureté ≥ 99,0 % en poids et d'une teneur en acétate d'iso-butyle de ≤ 1 000 ppm en poids.

17. Procédé selon l'une quelconque des revendications précédentes, **caractérisé en ce qu'**on met en œuvre la transformation en présence de quantités efficaces d'un stabilisant ou de plusieurs stabilisants.

18. Procédé selon l'une quelconque des revendications précédentes, **caractérisé en ce que** le niveau de liquide dans le réacteur est réglé au moyen du volume de retour de la phase aqueuse.

19. Procédé selon l'une quelconque des revendications précédentes, **caractérisé en ce que** pour le démarrage du procédé, l'ester de butyle d'acide acrylique correspondant ou un mélange de réaction correspondant contenant l'ester de butyle d'acide acrylique est chargé dans le réacteur.

20. Procédé selon l'une quelconque des revendications précédentes, **caractérisé en ce qu'**au moins un stabilisant est à chaque fois présent aussi bien dans la colonne de rectification que dans la phase organique résultant après l'élimination par distillation, lequel se dissout, en des proportions efficaces, aussi bien dans la phase aqueuse que dans la phase organique.
